# EUROPÄISCHE PATENTSCHRIFT

(11) **EP 4 102 162 B1**
(45) Veröffentlichungstag und Bekanntmachung des Hinweises auf die Patenterteilung: **11.11.2026**
(21) Anmeldenummer: 22178100.8
(22) Anmeldetag: 09.06.2022
(51) Int. Cl.: B62D 33/04, F25D 29/00, G01K 1/024, G01K 3/00, G07C 9/00

(54) **TRANSPORTBEHÄLTER ZUR VERWENDUNG IN EINEM TELEMETRIE-THERMOTRANSPORTSYSTEM**
TRANSPORT CONTAINER FOR USE IN A TELEMETRY THERMOTRANSPORT SYSTEM
CONTENEUR DE TRANSPORT DESTINÉ À ÊTRE UTILISÉ DANS UN SYSTÈME DE TRANSPORT THERMIQUE TÉLÉMÉTRIQUE

(30) Priorität: 09.06.2021 DE 102021114844
(43) Veröffentlichungstag der Anmeldung: 14.12.2022
(73) Patentinhaber: apocourier GmbH, 70839 Gerlingen (DE)
(72) Erfinder: Bordt, Oliver, 71272 Renningen (DE); Hopf, Katrin, 74354 Besigheim (DE)
(74) Vertreter: Spachmann, Holger

(56) Entgegenhaltungen:
- DE-A1- 102014 005 458
- DE-A1- 19 958 683
- US-A1- 2005 232 747
- US-A1- 2007 227 913
- US-A1- 2017 286 905

## Beschreibung

Die Erfindung betrifft einen Transportbehälter und ein Telemetrie-Thermotransportsystem mit einem Transportbehälter für den Transport von Medikamenten, pharmazeutischen Präparaten oder biologisch/chemisch sensitiven Substanzen.

### STAND DER TECHNIK

Aus dem Stand der Technik sind gattungsgemäße Transportsysteme bekannt, die unter definierbaren Transportbedingungen, insbesondere unter einer definierbaren Transportatmosphäre in einem spezifischen Temperaturbereich, Luftfeuchte und unter Kontrolle von Lage und mechanischen Einwirkungen wie Stößen und Erschütterungen, einen sicheren Transport von sensitiven Substanzen wie Medikamenten oder pharmazeutischen Präparaten, aber auch biologischen, chemischen Substanzen ermöglichen.

So zeigt die WO 2020 263 710 A1 eine portable Kühleinheit, welche unter anderem dafür geeignet ist, Medikamente zu transportieren. Dabei kann die Position des Behälters mittels einer App überwacht werden. Ferner können dem Nutzer Warnungen per App mitgeteilt werden, wenn bspw. der Kühlmitteleinlass verstopft ist.

Die WO 2019 178 152 A1 ist auf einen portablen Behälter zum Transportieren von onlinebestellten Produkten gerichtet. Der Behälter weist dabei eine Kontrolleinheit auf, die die Tür des Behälters verschließt bzw. freigibt sowie eine Temperaturkontrolleinheit, die die Temperatur innerhalb des Behälters regelt. Die Kontrolleinheit kann dabei mittels einer drahtlosen Verbindung mit einem Server verbunden werden. Die Kontrolleinheit wird mittels eines Codes gesteuert, welcher nur der Empfänger der Lieferung kennt.

Die CN 106 016 884 zeigt einen Behälter zum Transportieren von Impfstoffen. Dabei kann der Transportraum des Behälters mittels eisgekühltem Wasser in einem vorbestimmten Temperaturbereich gekühlt werden, in dem eisgekühltes Wasser aus einem Vorrat um den Transportraum geleitet wird. Dies kann mittels eines Temperatursensors und eines Ventils gesteuert werden. Dabei kann das eisgekühlte Wasser in den Vorrat nachgefüllt werden, ohne den Transportraum zu öffnen. Der Behälter ist nicht verschließbar.

Die US 2017/286905 A1 offenbart ein Telemetrie-Thermotransportsystem für den Transport von Medikamenten oder ähnlichem. Das System umfasst einen Transportbehälter, der einen kühlbaren/erwärmbaren sowie mechanisch verschließbaren Warenaufnahmebereich aufweist. Zudem ist eine Sensoreinheit zur Überwachung der Klimabedingungen in dem Transportbehälter vorgesehen. Der Transportbehälter ist über ein Netzwerk mit einer Fernsteuereinrichtung gekoppelt, die dazu ausgebildet ist, die Sensordaten abzufragen und ein steuerbares Verriegeln oder Entriegeln des Transportbehälters zu bewirken

Die DE 10 2014 005458 A1 offenbart eine transportable Kühlbox zum Anliefern von Lebensmitteln. Die Kühlbox umfasst einen isolierten Aufnahmebereich, welcher mittels eines Kühlelements und eines Stromanschlusses temperierbar ist. Der Aufnahmebereichen ist dabei durch einen Deckel verschließbar. Weiterhin ist vorgesehen, dass das Öffnen und Schließen der Kühlbox insbesondere nur mittels einer passenden Chipkarte, einem Schlüssel, einem Code oder ähnlichem möglich ist.

Die US 2007/227913 A1 offenbart ein Telemetrie - Transportsystem mit einem Transportbehälter und einer Fernsteuereinrichtung. Der Transportbehälter ist zum Transport und zur Lagerung von pharmazeutischen Artikeln ausgebildet und dazu insbesondere gezielt temperierbar, wobei unter anderem eine Sensoreinheit zur Kontrolle der Temperatur vorgesehen ist. Das Öffnen und Schließen des Transportbehälters ist dabei durch die Fernsteuereinrichtung steuerbar, wobei unter anderem eine Zugangskontrolle durch eine Authentifizierung mittels eines Smartphones oder ähnlichem stattfinden kann.

Die US 2005/232747 A1 offenbart einen gesicherten Transportbehälter zum Transport diverser Waren. Der Transportbehälter weist verschiedene Sensoren auf, mittels welchem unter anderem eine Kontrolle des Klimas im Inneren des Transportbehälters ermöglicht ist. Zudem weist der Transportbehälter einen Sicherungsmechanismus auf, mittels welchem ein unkontrolliertes Öffnen bzw. Schließen des Transportbehälters vermeidbar ist. Insbesondere ist zur Kontrolle des Zugriffs auf den Transportbehälter der Sicherungsmechanismus über ein Netzwerk mit einer Fernsteuereinrichtung verbunden.

Ein weiteres Dokument aus dem Stand der Technik ist DE19958683A1.

Es ergibt sich das Problem, dass beispielsweise nur der Apotheker das Sperren bzw.

Freigeben der Tür des Behälters regeln kann. Es ist weiterhin keine Möglichkeit bekannt, den Zugang zum gekühlten Fach zu kontrollieren, sodass eine Zugangskontrolle mit bekannten Lösungen nicht erfolgen kann.

Aufgabe ist es daher, die genannten Nachteile zu überwinden. Weiterhin ist es Aufgabe der vorliegenden Erfindung, ein temperierbares Transportsystem zu schaffen, das von der Ferne aus überwacht und kontrolliert werden kann.

Diese Aufgaben werden durch ein Telemetrie-Thermotransportsystem, ein Transportbehälter und eine Fernsteuereinrichtung nach den folgenden Ansprüchen gelöst. Vorteilhafte Ausbildungen der Erfindung sind Gegenstand der Unteransprüche.

### OFFENBARUNG DER ERFINDUNG

Die folgende Erfindung ist im unabhängigen Anspruch 1 offenbart. Weitere Ausführungsbeispiele sind in den abhängigen Ansprüchen offenbart.

Es wird ein Telemetrie-Thermotransportsystem nach Anspruch 8 für den Transport von Medikamenten, pharmazeutischen Präparaten, biologisch/chemisch sensitiven Substanzen oder dergleichen, umfassend zumindest einen Transportbehälter mit einem thermisch konfigurierbaren und mechanisch verriegelbaren Warenaufnahmebereich und eine Behältersteuereinrichtung, umfassend eine Sensoreinheit zur zumindest Temperaturüberwachung des Warenaufnahmebereichs und eine Verschlusseinheit zum steuerbaren Ver- bzw. Entriegeln des Warenaufnahmebereichs, und eine Fernsteuereinrichtung vorgeschlagen.

Erfindungsgemäß ist die Fernsteuereinrichtung über ein Netzwerk mit der Behältersteuereinrichtung verbunden ist, um Sensortelemetriedaten der Sensoreinheit abzufragen und ein steuerbares Verriegeln oder Entriegeln des Warenaufnahmebereichs durch die Verschlusseinheit zu bewirken.

Erfindungsgemäß wird vorgesehen, dass die Behältersteuereinheit auswechselbar in dem Transportbehälter aufnehmbar ist. Somit kann ein Energiespeicher einer Behältersteuereinheit getrennt aufgeladen werden, und je nach Art und Größe der zu transportierenden Ware in unterschiedliche Transportbehälter eingesetzt werden.

Unter Sensortelemetriedaten werden physikalische oder chemische, von Sensoren erfassbare Sensordaten, verstanden, die Transportbedingungen von in dem Warenaufnahmebereich befindlichen Transportwaren beeinflussen. Dies können insbesondere Temperatur, aber auch Luftfeuchte, Luftdruck, Atmosphärenzusammensetzung, Beschleunigung bzw. Erschütterung, Lage bzw. Ausrichtung oder Neigung zur Erdoberfläche, Lichteinwirkung bzw. Helligkeit sein, die während des Transports die im Warenaufnahmebereich befindlichen Waren betreffen. Auch die aktuelle Position des Transportbehälters kann über satellitengestützte Positionserkennung wie GPS, GLONASS oder GALILEO erfasst werden.

Die Behältersteuereinheit kann beispielsweise in Form eines Einplatinenrechners wie Raspberry Pi oder ähnliches, als Smartphone oder als dedizierte Elektronikplatine mit vordefinierbarer Programmierung als Embedded System ausgebildet sein. Insbesondere kann die Behältersteuereinheit ein Sensorinterface zu einem oder mehreren Sensoren und eine Aktuatorinterface zu einem Verschlussaktuator einer Verschlusseinheit, beispielsweise einem Verschlussmotor oder einem Verschlussmagneten, aufweisen. Die Behältersteuereinheit umfasst einen wiederaufladbaren und bevorzugt austauschbaren Energiespeicher, insbesondere Akku, der beispielsweise über einen Anschluss, bevorzugt USB-Anschluss, aufladbar sein kann. Über den Anschluss kann auch eine Änderung der Programmierung vorgenommen werden.

Die Verschlusseinheit kann als Stellaktuator einen Verschlussschieber eines Schlosses, der eine Öffnungsabdeckung, beispielsweise eine gelenkig angebrachte Tür des Warenaufnahmebereichs, beinhaltet, ausgebildet sein.

Die Fernsteuereinrichtung kann insbesondere als APP oder Software auf einem Mobilgerät wie Tablet, Smartphone, Notebook oder einem PC oder als Cloudanwendung ausgeführt sein, zu dem ein Sender der zu transportierten Waren Zugang hat.

In einer vorteilhaften Weiterbildung kann das Netzwerk ein Drahtlosnetzwerk sein, insbesondere ein Mobilfunknetzwerk, ein WLAN-Netzwerk und/oder ein NFC-Netzwerk, insbesondere eine Bluetooth-Verbindung oder RFID-Verbindung nutzen. So kann beispielsweise eine Verbindung der Behältersteuereinrichtung mit der Transportsteuereinrichtung mittels Bluetooth eingerichtet sein, und eine Verbindung zwischen Fernsteuereinrichtung und Transportsteuereinrichtung mittels Mobilfunknetz, insb. 4G oder 5G oder WLAN eingerichtet sein. Alternativ kann die Behältersteuereinrichtung über Mobilfunknetz oder WLAN unmittelbar mit der Fernsteuereinrichtung kommunizieren. Dabei kann vorteilhaft eine Internetverbindung über das Mobilfunknetz oder Bluetooth/WLAN zur Datenkommunikation genutzt werden.

In einer vorteilhaften Weiterbildung kann eine Transportsteuereinrichtung umfasst sein, die im Netzwerk kommunikativ zwischen Fernsteuereinrichtung und Behältersteuereinrichtung zwischengeschaltet oder parallelgeschaltet sein kann, und beim Transport räumlich benachbart zum Transportbehälter verbleiben kann. Die Transportsteuereinrichtung kann beispielsweise als APP auf einem mobilen Internetendgerät wie einem Smartphone oder Tablet eines Boten bzw. Pakettransporteurs ausgebildet sein. Diese wird somit mit dem Transportbehälter mittransportiert und kann als Informationsschnittstelle zwischen Fernsteuereinrichtung und Transportbehälter dienen, sowie eine Sprach- oder Videokommunikation zwischen Kunde und/oder Bote und Absender bereitstellen, beispielsweise eine Unterweisung eines Apothekers an einen Patienten zur Einnahme eines Medikamentes vermitteln.

In einer vorteilhaften Weiterbildung der vorgenannten Ausführungsform kann die Transportsteuereinrichtung eine Kommunikationseinheit umfassen, über die eine, vorzugsweise bidirektionale, Kommunikation zwischen der Behältersteuereinrichtung mit der Fernsteuereinrichtung bereitstellen kann, und bevorzugt eine Sensoreinheit insbesondere mit einem Positionssensor umfassen kann. Die Transportsteuereinrichtung kann vorteilhaft sowohl mit der Behältersteuereinrichtung und der Fernsteuereinrichtung kommunizieren. Die Transportsteuereinrichtung kann in Kombination mit der Behältersteuereinrichtung ein Entriegeln des Warenaufnahmebereichs bewirken, vorteilhaft unter Anwendung eines kryptographischen Sicherheitssystems.

In einer vorteilhaften Weiterbildung kann die Behältersteuereinrichtung mit der Fernsteuereinrichtung und/oder mit der Transportsteuereinrichtung über einen QR-Code und/oder ein NFC/RFID-Tag koppelbar sein. Hierdurch ist eine Verbindung, insbesondere eine kryptographisch verschlüsselte Verbindung zwischen Behältersteuereinrichtung und Fernsteuereinrichtung, ggf. über die Transportsteuereinrichtung etablierbar.

In einer vorteilhaften Weiterbildung kann die Fernsteuereinrichtung eingerichtet sein, Parameter zum selbsttätigen und/oder fernsteuerbaren Verriegeln und/oder Entriegeln der Verschlusseinheit in der Behältersteuereinrichtung zu konfigurieren, insbesondere Parameter auf Basis von Sensorwerten zu konfigurieren. Diese Parameter können Sensorparameter wie die Beibehaltung oder das Verlassen vordefinierbarer Sensorwerte, beispielsweise Temperaturwerte oder Erschütterungswerte, sein. Auf Basis der Parameter kann ein Entriegeln der Verschlusseinheit ermöglicht oder verweigert werden. Weiterhin kann als Parameter ein kryptographischer Schlüssel, beispielsweise ein Schlüssel eines Kunden, der eine Ware auf Basis eines Lieferscheins bzw. ein Medikament auf Basis eines elektronischen Rezeptes zugewiesen bekommen hat, sein. Die Fernsteuereinrichtung kann im Versendeprozess die Parameter vorgeben, unter denen eine Entriegelung beim Kunden ermöglicht wird.

Es wird ein Transportbehälter nach Anspruch 1 zur Verwendung in einem vorgenannten Telemetrie-Thermotransportsystem vorgesehen. Der Transportbehälter nach Anspruch 1 umfasst einen thermisch konfigurierbaren und mechanisch verriegelbaren Warenaufnahmebereich und eine Behältersteuereinrichtung, umfassend eine Sensoreinheit zur zumindest Temperaturüberwachung des Warenaufnahmebereichs und eine Verschlusseinheit zum steuerbaren Verriegeln bzw. Entriegeln des Warenaufnahmebereichs.

Erfindungsgemäß ist die Behältersteuereinheit auswechselbar in dem Transportbehälter aufnehmbar. Somit kann ein Energiespeicher einer Behältersteuereinheit getrennt aufgeladen werden, und je nach Art und Größe der zu transportierenden Ware in unterschiedliche Transportbehälter eingesetzt werden.

In einer vorteilhaften Weiterbildung kann im Transportbehälter zum Temperieren des Warenaufnahmebereichs zumindest ein Kühl- und/oder Heizelement auswechselbar aufnehmbar sein. Als Kühlelement können beispielsweise ein oder mehrere auswechselbare Kühlakkus dienen, als Heizelement ein oder mehrere chemische Thermopads, auch ist ein aktives, strombetriebenes Kühl- oder Heizelement denkbar, das beispielsweise über die Energieversorgung der Behältersteuereinheit mit Strom versorgt werden kann.

In einer vorteilhaften Weiterbildung kann im Transportbehälter die Sensoreinheit des Weiteren zumindest Sensoren für Lage, Position, Beschleunigung, Helligkeit, Atmosphärenzusammensetzung, Atmosphärendruck und/oder Luftfeuchtigkeit umfassen. Es können sowohl Atmosphärensensoren für Temperatur, Druck oder Feuchte, wie auch Mechanische für Lage oder Beschleunigung oder Photosensoren für Helligkeit umfasst sein, die die Transportbedingungen empfindlicher Waren überwachen können.

In einer vorteilhaften Weiterbildung kann im Transportbehälter die Verschlusseinheit des Transportbehälters durch einen optisch darstellbaren QR-Code und/oder ein Bluetooth/RFID/NFC-Signal verriegelbar oder entriegelbar sein, insbesondere im Zusammenwirken mit der Transportsteuereinrichtung und/oder der Fernsteuereinrichtung verriegelbar oder entriegelbar sein. Somit kann mittels eines vom Boten oder vom Kunden dargestellten QR-Codes über eine Kamera, die beispielsweise in einer Transportsteuereinrichtung oder in der Behältersteuereinrichtung integriert wird, ein Entriegeln der Verschlusseinheit bewirkt werden. Der QR-Code kann dazu vorteilhaft einen kryptographischen Schlüssel beinhalten. Auch kann ein entsprechendes Bluetooth oder RFID/NFC-Signal ein Entriegeln bewirken, ebenfalls kryptographisch gesichert.

In einer vorteilhaften Weiterbildung kann im Transportbehälter die Behältersteuereinrichtung eine Kryptographieeinheit, bevorzugt eine schlüsselbasierte Krypthographieeinheit, zur Freigabe eines Verriegelns oder Entriegelns der Verschlusseinheit auf Basis einer Schlüsselkommunikation mit der Fernsteuereinrichtung, der Transportsteuereinrichtung und/oder eines empfängerseitigen Schlüssels umfassen. So kann mittels eines privaten und öffentlichen Schlüssels ein manipulationssicherer Transport einer verderblichen oder toxischen Ware gewährleistet und eine Fehlnutzung der Ware verhindert werden.

In einer vorteilhaften Weiterbildung kann im Transportbehälter die Behältersteuereinrichtung eine Speichereinheit zum Aufzeichnen von Sensor- und Aktuatordaten zumindest vom Verriegeln des Warenaufnahmebereichs bis zum nachfolgenden Entriegeln des Warenaufnahmebereichs umfassen. Durch ein Datenlogging kann manipulationssicher die Transportbedingungen, ggf. auch der Routenverlauf des Transportes aufgezeichnet und bevorzugt in einer Cloud gespeichert oder von der Fernsteuereinrichtung abgefragt und ausgewertet werden.

In einer vorteilhaften Weiterbildung kann im Transportbehälter die Behältersteuereinrichtung eine Anzeigeeinrichtung zur optischen und/oder akustische Anzeige von Informationen, insbesondere eines QR-Codes, einer Statusmitteilung und/oder Warnmeldung umfassen. So kann bei Verlassen eines Temperaturbereichs, Überschreiten einer Transportzeit oder Umstürzen der Ware eine Warnmeldung ausgegeben werden. Gleichfalls kann ein Auslesen eines QR-Codes z.B. durch eine Kundensteuereinrichtung oder eine Transportsteuereinrichtung zur Identifikation der Ware und ggf. zum Entriegeln der Verschlusseinheit genutzt werden.

In einem weiteren nebengeordneten Aspekt wird eine Fernsteuereinrichtung zur Verwendung in einem vorstehend beschriebenen Telemetrie-Thermotransportsystem vorgeschlagen. Die Fernsteuereinrichtung ist mit einer Behältersteuereinrichtung und/oder mit einer Transportsteuereinrichtung koppelbar, um direkt oder indirekt in der Behältersteuereinrichtung umfasste Sensoren der Sensoreinheit auszulesen und die in der Behältersteuereinrichtung umfasste Verschlusseinheit zu steuern. Im Versendeprozess kann der Versender Typ und Menge der Waren in dem Transportbehälter erfassen und Parameter für den Transport und zur Freigabe einer Entriegelung am Bestimmungsort des Kunden festlegen. Ein Entriegeln kann autark, d.h. ohne Zutun der Fernsteuereinrichtung erfolgen, z.B. beim Einhalten aller Parameter wie Kundenidentifikation, Einhalten von Sensorparametern, Einhalten einer Transportzeit, etc. oder kann ferngesteuert durch explizite Freigabe durch die Fernsteuereinrichtung, z.B. nach einer Unterweisung bzgl. des Umgangs mit der Ware, z.B. eine Dosierungsanleitung durch einen Apotheker, mittels Sprach- oder Videokommunikation, entweder über eine Transportsteuereinrichtung oder die Behältersteuereinrichtung erfolgen. Auch kann ein Abspielen eines Videos oder die Darstellung eines Textes als Voraussetzung für die Entriegelung festgelegt werden.

So kann in einer, beispielsweise sowie nachfolgend als apocourier-Lösung bezeichneten, Ausführungsform ein von einem Apotheker jederzeit kontrollierbarer Botendienst ermöglicht werden, welcher die jederzeitige Weisungshoheit des Apothekers technisch und ohne Ausübung eines arbeitsrechtlichen Direktionsrechtes ermöglicht und die Möglichkeit des Nachweises der Einhaltung von Qualitätsvorgaben enthält.

Somit kann in einer Ausführungsform folgendes umfasst sein:
a) ein Bote mit Transportsteuereinrichtung und
b) eine Apotheker Software-Applikation als Fernsteuereinrichtung,
wobei zwischen a) und b) insbesondere ein über das Internet und/oder einen Server als Zwischenspeicherung laufender Datenaustausch erfolgt.

In einer weiteren Ausführungsform kann in c) ein mit a) und b) über eine Kommunikationsschnittstelle (z.B. Bluetooth) verbundener Transportbehälter folgendes umfassen:
aa) einer Kunststoffbox, welche insbesondere einen Schacht für eine Kühleinheit, z.B. einem oder mehreren Kühlakkus, und/oder einen Schacht für eine herausnehmbare Behältersteuereinrichtung und/oder einen über eine Behältersteuereinrichtung mit Verschlusseinheit und Schlossmechanik verschließbaren Warenaufnahmebereich als Schacht in der Kunststoffbox umfasst.

Die herausnehmbare Behältersteuereinrichtung umfasst eine Sensoreinheit zur zumindest Temperaturüberwachung des Warenaufnahmebereichs und eine Verschlusseinheit zum steuerbaren Verriegeln bzw. Entriegeln des Warenaufnahmebereichs, und kann in einer Ausführungsform Sensoren, insbesondere für Luftfeuchtigkeit, Erschütterung etc., und/oder Aktoren. insbesondere ausgebildet als zumindest eine Diode und/oder zumindest ein Signalton, zumindest ein Display und oder zumindest eine Anzeige umfassen.

In einer weiteren Ausführung kann alternativ oder zusätzlich eine Speichereinheit, insbesondere ausgebildet als Datenlogger und/oder insbesondere auslesbar über Applikation der Fernsteuereinrichtung und/oder Transportsteuereinrichtung, umfasst sein.

In einer weiteren Ausführung kann alternativ oder zusätzlich eine Lösung zur einfachen Herstellung einer Verbindung zwischen Softwareapplikation der Fernsteuereinrichtung und/oder Transporteinrichtung und der Behältersteuereinrichtung des Transportbehälters über einen individuellen NFC Tag Code, welcher insbesondere die Verbindungsdaten für die Bluetooth Verbindung enthält, ermöglicht werden.

Das Telemetrie-Thermotransportsystem, auch bezeichnet als Apocourier Box, ermöglicht die unkomplizierte und zuverlässige Auslieferung von Arzneimitteln. Abgestimmt auf Apotheker und ihre Kunden. Einfach und intuitiv steuerbar mit einer sogenannten Apocourier App als Fernsteuereinrichtung. Dieselben Vorteile gelten für den Transportbehälter sowie die Fernsteuereinrichtung.

Innerhalb des Telemetrie-Thermotransportsystem kann der Transportbehälter die auszuliefernden Arzneimittel kühlen und überwachen. Insbesondere kann zumindest eine Abweichung von vorab eingestellten Parametern direkt an den Apotheker durch das Netzwerk übermittelt werden. Zusätzlich kann der Bote durch die Transportsteuereinrichtung alarmiert werden. Die Einhaltung pharmazeutischer Qualitätskriterien kann nachvollziehbar durch die Speichereinheit dokumentiert und archiviert werden.

Der Transportbehälter ist, bevorzugt elektronisch, bis zum Eintreffen beim Kunden/Patienten verriegelt. Durch das Telemetrie-Thermotransportsystem ist daher voreilhaft eine durchgehende Weisungshoheit und Kontrolle des Apothekers gewährleistet - insbesondere bis vor die Haustür des Kunden. Die Auslieferung kann daher jederzeit durch eine zuverlässige und abgesicherte Apothekerhand erfolgen.

Der Transportbehälter kann in der Apotheke elektronisch und sicher verriegelt werden und kann daher vorteilhaft erst vor Ort beim Kunden entriegelt werden. Pharmazeutische Fragen des Kunden können über eine integrierte Sprach- oder Videochat-Funktion unkompliziert und kompetent durch die Apotheke beantwortet werden.

Vorteilhafterweise ermöglicht das Telemetrie-Thermotransportsystem eine einfache und schnelle Lieferung, insbesondere mit eigenen Fahrern und/oder anderen Service-Anbietern vor Ort.

Insbesondere durch Kühlakkus kann die Kühlkette für Arzneimittel bis zur Auslieferung sichergestellt werden. In einer Ausführungsform kann ein Alarmsystem die eingestellten Temperaturparameter überwachen und/oder dokumentieren. Weiterhin kann der Apotheker über die Fernsteuereinrichtung und/oder den Boten durch die Transportsteuereinrichtung und/oder die Behältersteuereinrichtung, insbesondere bei einer Überschreitung, gewarnt werden.

Der Apotheker kann daher vorteilhaft den Prozess steuern und die Auslieferung mit der intuitiven und einfach anzuwendenden APP überwachen, beispielsweise auf seinem Smartphone, Tablet oder PC. Dies kann voreilhaft und insbesondere kontinuierlich von der Disposition bis zur Auslieferung erfolgen. Der Apotheker kann daher jederzeit eingreifen und eine qualitativ hochwertige Auslieferung der Arzneimittel sicherstellen.

Insbesondere kann eine Umsetzung von regulatorischen Anforderungen einfach und sicher erfolgen. Dies betrifft beispielsweise eine Temperaturkontrolle. Diese erfolgt insbesondere über zumindest einen Temperatursensor und/oder einen Temperaturlogger.

Weiterhin kann eine Kontrolle sowie Weisungshoheit des Apothekers durch technische Mittel statt arbeitsrechtliches Direktionsrecht erfolgen. Dies gilt insbesondere für eine Entsperrkontrolle, für eine jederzeitige Abrufbarkeit von Sensordaten sowie für eine Kommunikation und Normierung von Auslieferungsvorgängen über eine Apotheker-App.

Weiterhin erfolgt eine Sicherstellung der Qualität zu jeder Zeit. Dies kann insbesondere durch eine Passivkühlung, einen Temperaturalarm und/oder einen Austausch von Kühlakkus ohne Entsperrung ermöglicht werden.

Weiterhin kann eine Beteiligung von pharmazeutischem Personal bei der Auslieferung sowie eine persönliche Betreuung durch den Apotheker, insbesondere durch die Fernsteuereinrichtung, sichergestellt werden. Dies kann über eine Videofunktion bzw. über Auslieferungsanweisungen über die Fernsteuereinrichtung erfolgen.

Demnach kann eine Dokumentation sowie eine Steuerung der Auslieferung gemäß gesetzlichen Anforderungen sichergestellt werden.

Insbesondere erfolgt dies über eine Workflowsteuerung über eine Applikation und/oder über eine auditsichere Dokumentation über Mustertexte zur Erfüllung der rechtlichen Anforderungen.

Folglich können mit dem Thermostattransportsystem Anforderungen umgesetzt werden, die über einen gewöhnlichen Versandhandel oder einen gewöhnlichen Botendienst ohne den Einsatz von pharmazeutisch ausgebildeten Boten nicht erfolgen kann.

Mit dem Telemetrie-Thermotransportsystem wird Sorgfalt und Expertise einer Apotheke auch innerhalb der Lieferkette eines Botendienstes bzw. Paketzustelldienstes mit bestmöglicher Qualität gewährleistet.

Das Telemetrie-Thermotransportsystem kann auch im Hinblick auf Anonymisierung, Datensicherheit, zuverlässige Transportsicherheit und/oder Datenschutz höchste Standards erreichen.

### ZEICHNUNGEN

Weitere Vorteile ergeben sich aus der beiliegenden Zeichnungsbeschreibung. In den Zeichnungen sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen, die den folgenden Ansprüchen entsprechen.

Durch die Zeichnungen werden einzelne Ausführungsformen einer möglichen Anwendung des Telemetrie-Thermotransportsystems als sogenannte apocourier-Lösung für einen Transport von Medikamenten von einem Apotheker zu einem Patienten, beispielsweise unter Nutzung einer Zustelllogistik, wie insbesondere einem Boten, bzw. Paketdienst oder Lieferservice, dargestellt.

Es zeigen:
- **Fig. 1**: eine Ausführungsform eines Telemetrie-Thermotransportsystems in einer Außenansicht;
- **Fig. 2**: das Telemetrie-Thermotransportsystem aus Fig. 1 in einem geöffneten Zustand;
- **Fig. 3**: eine weitere Ausführungsform eines Telemetrie-Thermotransportsystems;
- **Fig. 4**: eine weitere Ausführungsform eines Telemetrie- Thermotransportsystems;
- **Fig. 5**: eine weitere Ausführungsform eines Telemetrie-Thermotransportsystems;
- **Fig. 6**: eine Ausführungsform einer Behältersteuereinrichtung eines Telemetrie-Thermotransportsystems;
- **Fig. 7**: eine weitere Ausführungsform einer Behältersteuereinrichtung eines Telemetrie-Thermotransportsystems;
- **Fig. 8**: eine schematische Darstellung eines Ablaufs eines Transports von Gütern mit dem Telemetrie-Thermotransportsystem.

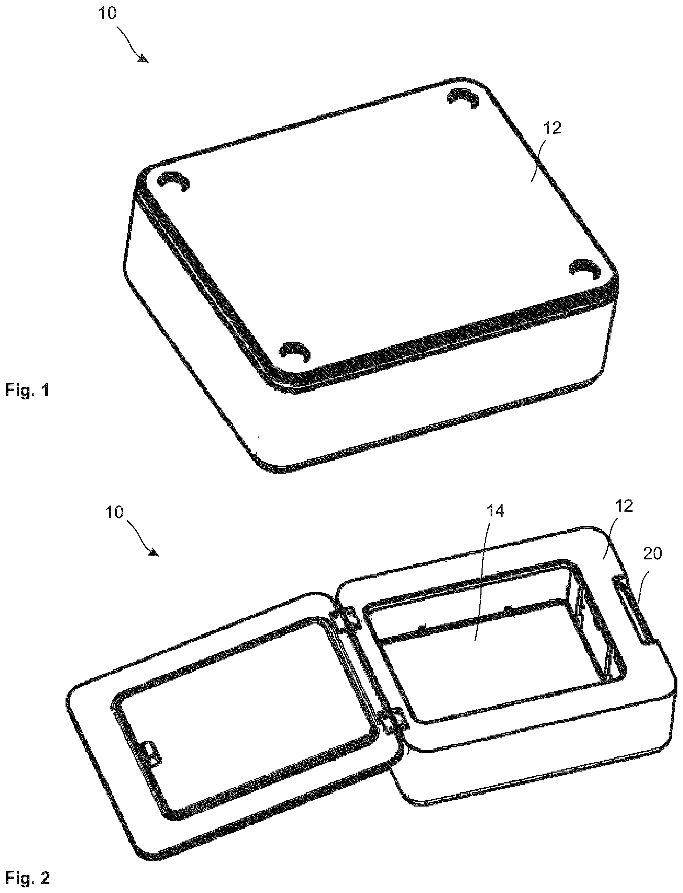

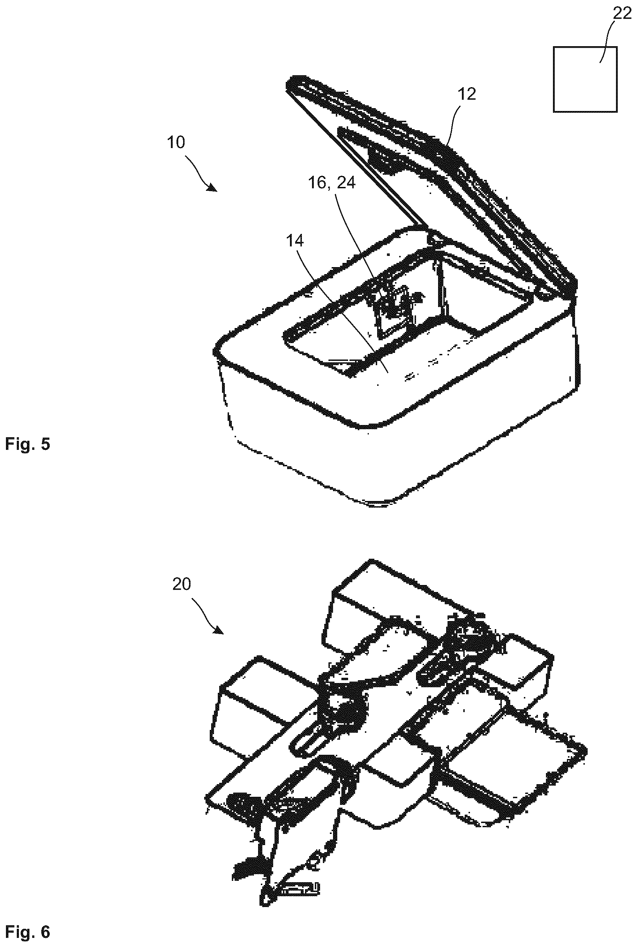

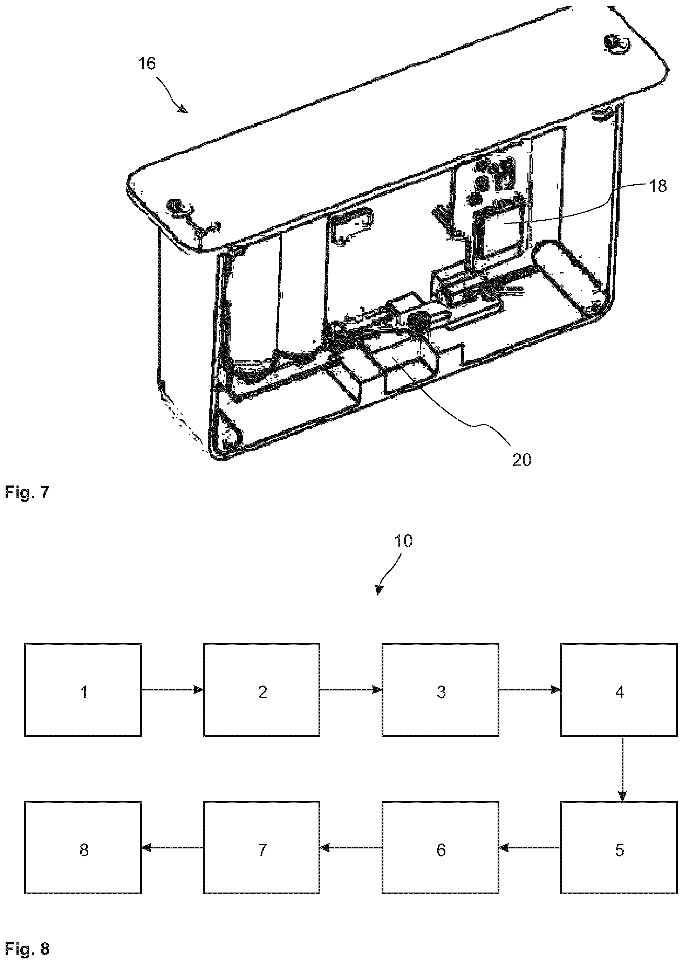

Fig. 1 zeigt eine Ausführungsform eines Telemetrie-Thermotransportsystems 10 in einer Außenansicht. Das Telemetrie-Thermotransportsystem 10 ist für den Transport von Medikamenten, pharmazeutischen Präparaten oder biologisch/chemisch sensitiven Substanzen ausgebildet. Es umfasst zumindest einen Transportbehälter 12 nach Anspruch 1 mit einem thermisch konfigurierbaren und mechanisch verschließbaren Warenaufnahmebereich 14 und eine Behältersteuereinrichtung 16. Die Behältersteuereinheit 16 umfasst eine Sensoreinheit 18 zur zumindest Temperaturüberwachung des Warenaufnahmebereichs, wie in Fig. 7 dargestellt. Weiterhin weist der Transportbehälter 12 eine Verschlusseinheit 20 zum steuerbaren Verriegeln bzw. Entriegeln des Warenaufnahmebereichs 14 auf, dargestellt in Fig. 2. Des Weiteren ist eine Fernsteuereinrichtung 22 umfasst, dargestellt in Fig. 5.

Die Abmessungen des Warenaufnahmebereichs 14 können 30 x 20 x 10 cm aufweisen. Ebenso kann die Grundfläche eine Größe DIN A 4 aufweisen. Die Seitenabmessungen liegen daher bevorzugt zwischen 10 und 35 cm. Andere Abmessungen sind ebenso denkbar.

Der Transportbehälter 12 kann zumindest ein Kühlfach aufweisen, in welchem zumindest ein Kühlakku gelagert werden kann. Vorteilhafterweise weist der Transportbehälter 12 zwei bis sechs, insbesondere vier Kühlfächer auf. Die Kühlakkus können unabhängig von der Verschlusseinheit 20 ausgetauscht werden. Dies bedeutet, dass ohne Öffnen der Verschlusseinheit 20 die Akkus ausgetauscht werden können.

Vorteilhafterweise ist der Transportbehälter 12 wasserundurchlässig ausgebildet. In einer Ausführungsform wird Kondenswasser der Kühlakkus aufgefangen, sodass es nicht in das Innere des Warenaufnahmebereichs 14 oder in Kontakt mit den Steuereinheiten 16, 24 kommt.

Durch die Form mit bevorzugt abgerundeten Ecken und Kanten ist der Transportbehälter 12 einfach zu desinfizieren. Weiterhein können mehrere, insbesondere baugleiche, Transportbehälter 12 einfach gestapelt werden. Dies kann insbesondere rutschsicher erfolgen. Die Transportbehälter 12 können aus einem ABS oder PMMA ausgebildet sein, sodass diese kratzunempfindlich sind. Weiterhin sind individuelle Beschriftungen oder Brandings und/oder Farbgebungen möglich.

Der Transportbehälter 12 kann beispielsweise über zumindest einen USB-Anschluss verfügen. Über diesen kann der Transportbehälter 12 beispielsweise aufgeladen werden.

Die Fernsteuereinrichtung 22 kann als Applikation (App) oder Software auf einem Mobilgerät wie Tablet, Smartphone, Notebook oder einem PC oder als Cloudanwendung ausgeführt sein, zu dem ein Sender der zu transportierten Waren Zugang hat. Über eine derartige App bzw. Applikation kann beispielsweise eine lückenlose Protokollierung der pharmazeutischen Kontrolle erfolgen und gespeichert werden. Dies kann beispielsweise eine ständige Kontrolle der Temperatur sein. In einer Ausführungsform kann ein Random 32 Byte Key mit Brut Force prevention für maximale digitale Sicherheit eingesetzt werden. Weiterhin kann beispielsweise ein dynamischer Sensor-Check mit preventive demolition detection (PDD) erfolgen. In einer weiteren Ausführung kann die Sicherheit durch abstrahierte lokale d.h. ohne ständige Datenübertragung an den Server durch passives ID Matching und aktivem Sandbox-Modus erfolgen. Weiterhin kann ein Symfony-based high-performance Data-Hub für Master Data Management und einfache Anbindung an ein ERP-System eingesetzt werden. Die Entwicklungen können unter Berücksichtigung einer API Layer Architektur erfolgen. Beispielsweise kann eine ständige Zugriffskontrolle durch eine JWT-Authentifizierung erfolgen. Die vorgenannten Beispiele können in Kombination oder einzeln eingesetzt werden.

Der Transportbehälter 12 kann dann beispielsweise als Kunststoffkofferbox mit drei verschiedenen Schächten bzw. Aufnahmebereichen ausgebildet sein. Einer der Schächte kann als sogenannte Technikbox zur Aufnahme der Steuereinrichtungen dienen, ein weiterer Schacht kann zur Aufnahme von zumindest einem Kühlakku ausgebildet sein. Ein dritter Schacht dient für die zu transportierenden Arzneimittel.

Fig. 2 zeigt das Telemetrie-Thermotransportsystem 10 aus Fig. 1 in einem geöffneten Zustand. In dieser Ansicht ist der Warenaufnahmebereich 14 erkennbar. Der Transportbehälter 12 nach Anspruch 1 weist einen Deckel auf, der über Scharniere angebracht ist.

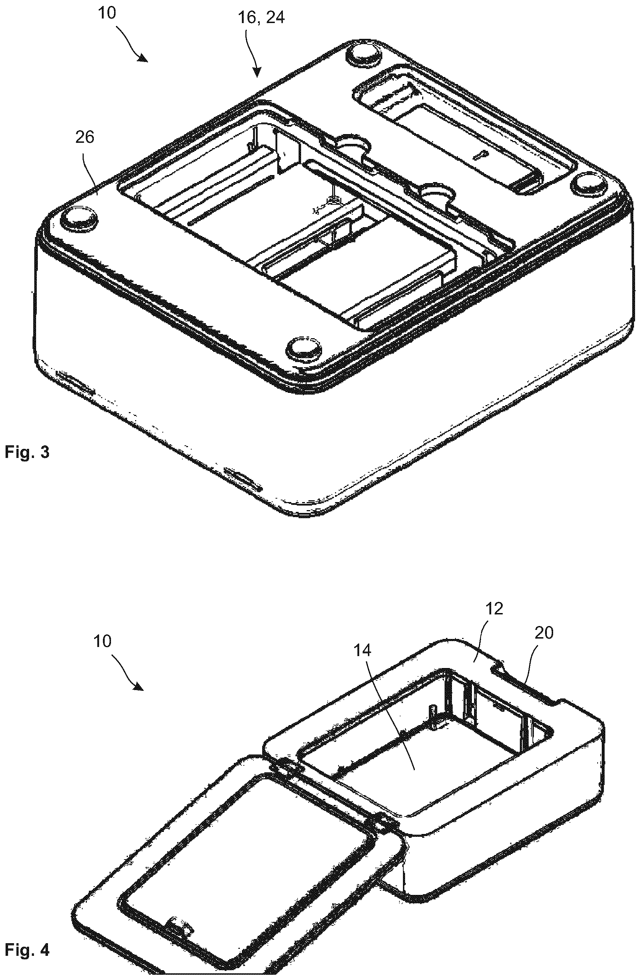

Fig. 3 zeigt eine weitere Ausführungsform eines Telemetrie-Thermotransportsystems 10. Die Darstellung ist von einer Bodenseite 26 gezeigt, wobei an der Bodenseite 26 eine Behältersteuereinrichtung 16 bzw. eine Transportsteuereinrichtung 24 erkennbar ist. Die Behältersteuereinrichtung 16 bzw. die Transportsteuereinrichtung 24 können in einem Bodenbereich oder in einem Seitenbereich in dem Transportbehälter 12 integriert sein.

Fig. 4 zeigt eine weitere Ansicht des Telemetrie-Thermotransportsystems 10 in einem geöffneten Zustand. In den Bereichen zwischen dem Warenaufnahmebereich 14 und den Außenkanten des Transportbehälters 12 können die Behältersteuereinrichtung 16 und/oder die Transportsteuereinrichtung 24 integriert sein.

Fig. 5 zeigt eine weitere Ausführungsform eines Telemetrie-Thermotransportsystems 10, wobei der Deckel nicht komplett geöffnet dargestellt ist. Des Weiteren ist die Fernsteuereinrichtung 22 schematisch dargestellt, welche unabhängig von dem Transportbehälter 12 nach Anspruch 1 bewegt werden kann.

Fig. 6 und Fig. 7 zeigen Ausführungsformen einer Behältersteuereinrichtung 16 eines Telemetrie-Thermotransportsystems 10. In Fig. 6 ist die Verschlusseinheit 20 mit einer mechanischen Verriegelung gezeigt. Diese ist in der Behältersteuereinrichtung 16 in Fig. 7 integriert. In Fig. 16 ist eine Platine inklusive einer Firmware umfasst. Weiterhin ist vorteilhafterweise zumindest ein Akku zur Stromversorgung umfasst. Des Weiteren kann eine elektronische Schlossmechanik mit einem Motor umfasst sein, die zur Regelung der Verschlusseinheit 20 ausgebildet ist. Weitere Aktoren, insbesondere optische und/oder akustische Aktoren, können Warnungen bei insbesondere Temperaturüberschreitungen ausgeben. Des Weiteren kann ein Logger umfasst sein, der Sensordaten über die Fernsteuereinrichtung 22 an den Apotheker übermitteln kann. Ebenso können zumindest ein Thermometer, ein Hygrometer, ein Schlüsselsensor, zumindest eine Schnittstelle, ein USB-C Anschluss und/oder ein Bluetooth und/oder ein NFC Empfänger, umfasst sein.

Durch die Behältersteuereinrichtung 16, die Transportsteuereinrichtung 24 sowie die Fernsteuereinrichtung 22 kann insbesondere eine durchgehende Weisungshoheit über einen Boten bis vor eine Haustüre eines Patienten erfolgen. Dies gewährleistet eine umfassende pharmazeutische Kontrolle über eine Auslieferung von beispielsweise Arzneimitteln. Dabei kann die Kühlung der Arzneimittel und Überwachung der Kühlkette in Echtzeit durchgeführt werden. Weiterhin kann eine Beratung von Patienten über beispielsweise eine Videofunktion, insbesondere wenn die Fernsteuereinrichtung 22 als Applikation ausgebildet ist, erfolgen. Es ist eine durchgehende Weisungshoheit über die Applikation auch ohne arbeitsrechtliches Direktionsrecht über technische Lösungen bereitgestellt. Insbesondere kann dabei eine Kooperation mit gewöhnlichen Bodendiensten erfolgen. Weiterhin kann durch die Applikation eine Limitierung von Retaxrisiken entstehen.

Insbesondere für ein Qualitätsmanagement sind die vorgenannten Merkmale von großem Vorteil. In jedem Fall kann das Thermotransportsystem 10 maßgeschneidert für unterschiedliche Apotheken vor Ort angepasst werden. Damit ist eine derartige Ausführung jedenfalls schneller als eine Versandapotheke. Vorteilhafterweise kann weiterhin durch den so bereitgestellten Botendienst ohne vorherige Abgabe einer Verordnung in der Apotheke eine Auslieferung erfolgen.

Fig. 8 zeigt eine schematische Darstellung eines Ablaufs eines Transports von Gütern mit dem Telemetrie-Thermotransportsystem 10. In Schritt 1 erfolgt eine Rezeptbestellung, wobei ein Apotheker einen Auftrag an einen bekannten oder unbekannten Boten vergibt. Dabei kann eine Auswahl nach der Priorität der Fahrstrecke erfolgen. In Schritt 2 wird das Medikament von der Apotheke in den Transportbehälter 12 verpackt. In Schritt 3 verschließt der Apotheker, insbesondere kryptographisch digital, mit einem in jedem Einzelfall neu generierten Schlüssel die Transportbehälter 12 und stellt beispielsweise einen Temperaturkorridor ein. Der Apotheker kann dabei die Übergabe an einen Kunden von einem vorherigem Videoanruf abhängig machen. Dies kann beispielsweise durch die Fernsteuereinrichtung 22, mithilfe einer Applikation, erfolgen. In Schritt 4 erhält der Bote den verschlossenen Transportbehälter 12 ohne Einsichtsmöglichkeit. In Schritt 5 erfolgt eine Fahrt zu einem Empfänger unter dauerhafter Kontrolle und Zugriff des Apothekers. Dabei kann der Apotheker die Auslieferung jederzeit stoppen und die Verschlusseinheit 20 sperren. In Schritt 6 erfolgt eine Auslieferung an den Kunden, wobei der Bote mit einer gesicherten und sofern notwendig gekühlten Ladung ankommt. In Schritt 7 kann ein Pre-Check, insbesondere der Temperaturkontrolle bzw. Identitätskontrolle, durch den Boten durchgeführt werden. In Schritt 8 erfolgt eine Übergabe und eine Öffnung der Verschlusseinheit 20 durch den Boten und durch den Patienten bzw. Kunden. Sollten hierbei Fragen entstehen so kann jederzeit über die Fernsteuereinrichtung 22, insbesondere einen Video-Anruf, der Apotheker zu einer weiteren Beratung hinzugezogen werden.

### Bezugszeichenliste

- 10: Telemetrie-Thermotransportsystem
- 12: Transportbehälter
- 14: Warenaufnahmebereich
- 16: Behältersteuereinrichtung
- 18: Sensoreinheit
- 20: Verschlusseinheit
- 22: Fernsteuereinrichtung
- 24: Transportsteuereinrichtung
- 26: Bodenseite

## Patentansprüche

1. Transportbehälter (12) zur Verwendung in einem Telemetrie-Thermotransportsystem (10) mit einer Fernsteuereinrichtung (22), wobei ein thermisch konfigurierbarer und mechanisch verschließbarer Warenaufnahmebereich (14) und eine Behältersteuereinrichtung (16), umfassend eine Sensoreinheit zur zumindest Temperaturüberwachung des Warenaufnahmebereichs (14) und eine Verschlusseinheit (20) zum steuerbaren Verriegeln bzw. Entriegeln des Warenaufnahmebereichs (14), umfasst ist, **dadurch gekennzeichnet, dass** die Behältersteuereinrichtung (16) auswechselbar in dem Transportbehälter aufnehmbar ist, wobei die Behältersteuereinrichtung (16) einen wiederaufladbaren Energiespeicher umfasst, sodass der Energiespeicher getrennt von dem Transportbehälter aufladbar ist und die Behältersteuereinrichtung in unterschiedliche Transportbehälter (12) einsetzbar ist.

2. Transportbehälter (12) nach Anspruch 1, **dadurch gekennzeichnet, dass** zum Temperieren des Warenaufnahmebereichs (14) zumindest ein Kühl- und/oder Heizelement auswechselbar aufnehmbar ist.

3. Transportbehälter (12) nach einem der vorhergehenden Ansprüche, **dadurch gekennzeichnet, dass** die Sensoreinheit des Weiteren zumindest Sensoren für Lage, Position, Beschleunigung, Lichteinwirkung, Atmosphärenzusammensetzung, Atmosphärendruck und/oder Luftfeuchtigkeit umfasst.

4. Transportbehälter (12) nach einem der vorhergehenden Ansprüche, **dadurch gekennzeichnet, dass** die Verschlusseinheit (20) durch einen optisch darstellbaren QR-Code und/oder ein Bluetooth/RFID/NFC-Signal verriegelbar oder entriegelbar ist, insbesondere im Zusammenwirken mit einer Transportsteuereinrichtung (24) und/oder der Fernsteuereinrichtung (22) verriegelbar oder entriegelbar ist.

5. Transportbehälter (12) nach einem der vorhergehenden Ansprüche, **dadurch gekennzeichnet, dass** die Behältersteuereinrichtung (16) eine Kryptographieeinheit, bevorzugt eine schlüsselbasierte Krypthographieeinheit, zur Freigabe eines Verriegelns oder Entriegelns der Verschlusseinheit (20) auf Basis einer Schlüsselkommunikation mit der Fernsteuereinrichtung (22), der Transportsteuereinrichtung (24) und/oder eines empfängerseitigen Schlüssels umfasst.

6. Transportbehälter (12) nach einem der vorhergehenden Ansprüche, **dadurch gekennzeichnet, dass** die Behältersteuereinrichtung (16) eine Speichereinheit zum Aufzeichnen von Sensor- und Aktuatordaten zumindest vom Verriegeln des Warenaufnahmebereichs (14) bis zum nachfolgenden Entriegeln des Warenaufnahmebereichs (14), umfasst.

7. Transportbehälter (12) nach einem der vorhergehenden Ansprüche, **dadurch gekennzeichnet, dass** die Behältersteuereinrichtung (16) eine Anzeigeeinrichtung zur optischen und/oder akustische Anzeige von Informationen, insbesondere eines QR-Codes, einer Statusmitteilung und/oder Warnmeldung umfasst.

8. Telemetrie-Thermotransportsystem (10) für den Transport von Medikamenten, pharmazeutischen Präparaten oder biologisch/chemisch sensitiven Substanzen, umfassend zumindest einen Transportbehälter (12) mit einem thermisch konfigurierbaren und mechanisch verschließbaren Warenaufnahmebereich (14) und einer Behältersteuereinrichtung (16), umfassend eine Sensoreinheit (18) zur zumindest Temperaturüberwachung des Warenaufnahmebereichs und eine Verschlusseinheit (20) zum steuerbaren Verriegeln bzw. Entriegeln des Warenaufnahmebereichs (14), und eine Fernsteuereinrichtung (22), wobei die Fernsteuereinrichtung (22) über ein Netzwerk mit der Behältersteuereinrichtung (16) verbunden ist, um Sensortelemetriedaten der Sensoreinheit (18) abzufragen und ein steuerbares Verriegeln oder Entriegeln des Warenaufnahmebereichs (14) durch die Verschlusseinheit (20) zu bewirken, **gekennzeichnet durch** eine Ausbildung des Transportbehälters (12) nach einem der Ansprüche 1 bis 7.

9. Telemetrie-Thermotransportsystem (10) nach Anspruch 8, **dadurch gekennzeichnet, dass** das Netzwerk ein Drahtlosnetzwerk ist, insbesondere ein Mobilfunknetzwerk, ein WLAN-Netzwerk und/oder ein NFC-Netzwerk, insbesondere eine Bluetooth-Verbindung oder RFID-Verbindung umfasst.

10. Telemetrie-Thermotransportsystem (10) nach Anspruch 8 oder 9, **dadurch gekennzeichnet, dass** eine Transportsteuereinrichtung (24) umfasst ist, die im Netzwerk kommunikativ zwischen Fernsteuereinrichtung (22) und Behältersteuereinrichtung (16) zwischengeschaltet oder parallelgeschaltet ist, und beim Transport räumlich benachbart zum Transportbehälter (12) verbleibt.

11. Telemetrie-Thermotransportsystem (10) nach Anspruch 10 **dadurch gekennzeichnet, dass** die Transportsteuereinrichtung (24) eine Kommunikationseinheit umfasst, über die eine, vorzugsweise bidirektionale, Kommunikation zwischen der Behältersteuereinrichtung (16) mit der Fernsteuereinrichtung (22) bereitstellbar ist, und bevorzugt eine Sensoreinheit insbesondere mit einem Positionssensor umfasst ist.

12. Telemetrie-Thermotransportsystem (10) nach einem der Ansprüche 8 bis 11, **dadurch gekennzeichnet, dass** die Behältersteuereinrichtung (16) mit der Fernsteuereinrichtung (22) und/oder mit der Transportsteuereinrichtung (24) über einen QR-Code und/oder ein NFC/RFID-Tag koppelbar ist.

13. Telemetrie-Thermotransportsystem (10) nach einem der Ansprüche 8 bis 12, **dadurch gekennzeichnet, dass** die Fernsteuereinrichtung (22) eingerichtet ist, Parameter zum selbsttätigen und/oder fernsteuerbaren Verriegeln und/oder Entriegeln der Verschlusseinheit (20) in der Behältersteuereinrichtung (16) zu konfigurieren, insbesondere Parameter auf Basis von Sensorwerten zu konfigurieren.

## Claims

1. Transport container (12) for use in a telemetry thermotransport system (10) having a remote control device (22), wherein a thermally configurable and mechanically closable goods-receiving region (14) and a container control device (16), comprising a sensor unit for at least temperature monitoring of the goods-receiving region (14) and a closure unit (20) for controllably locking or unlocking the goods-receiving region (14), are comprised, **characterized in that** the container control device (16) is configured to be exchangeably received in the transport container, wherein the container control device (16) comprises a rechargeable energy store, so that the energy store is chargeable separately from the transport container and the container control device is useable in different transport containers (12).

2. Transport container (12) according to claim 1, **characterized in that** at least one cooling and/or heating element is configured to be exchangeably received for temperature-controlling the goods-receiving region (14).

3. Transport container (12) according to any one of the preceding claims, **characterized in that** the sensor unit further comprises at least sensors for position, acceleration, exposure to light, atmospheric composition, atmospheric pressure and/or humidity.

4. Transport container (12) according to any one of the preceding claims, **characterized in that** the closure unit (20) is configured to be locked or unlocked by means of an optically displayable QR code and/or a Bluetooth/RFID/NFC signal, in particular in interaction with a transport control device (24) and/or the remote control device (22).

5. Transport container (12) according to any one of the preceding claims, **characterized in that** the container control device (16) comprises a cryptography unit, preferably a key-based cryptography unit, for enabling locking or unlocking of the closure unit (20) on the basis of key communication with the remote control device (22), the transport control device (24) and/or a receiver-side key.

6. Transport container (12) according to any one of the preceding claims, **characterized in that** the container control device (16) comprises a memory unit for recording sensor and actuator data at least from locking of the goods-receiving region (14) until subsequent unlocking of the goods-receiving region (14).

7. Transport container (12) according to any one of the preceding claims, **characterized in that** the container control device (16) comprises a display device for optical and/or acoustic display of information, in particular of a QR code, a status message and/or a warning message.

8. Telemetry thermotransport system (10) for transporting medicines, pharmaceutical preparations or biologically/chemically sensitive substances, comprising at least one transport container (12) having a thermally configurable and mechanically closable goods-receiving region (14) and a container control device (16), comprising a sensor unit (18) for at least temperature monitoring of the goods-receiving region and a closure unit (20) for controllably locking or unlocking the goods-receiving region (14), and a remote control device (22), wherein the remote control device (22) is connected to the container control device (16) via a network in order to query sensor telemetry data of the sensor unit (18) and to effect controllable locking or unlocking of the goods-receiving region (14) by the closure unit (20), **characterized by** a configuration of the transport container (12) according to any one of claims 1 to 7.

9. Telemetry thermotransport system (10) according to claim 8, **characterized in that** the network is a wireless network, in particular a mobile radio network, a WLAN network and/or an NFC network, in particular comprises a Bluetooth connection or RFID connection.

10. Telemetry thermotransport system (10) according to claim 8 or 9, **characterized in that** a transport control device (24) is comprised, which is communicatively interposed or connected in parallel in the network between the remote control device (22) and the container control device (16), and remains spatially adjacent to the transport container (12) during transport.

11. Telemetry thermotransport system (10) according to claim 10, **characterized in that** the transport control device (24) comprises a communication unit via which communication, preferably bidirectional communication, between the container control device (16) and the remote control device (22) is providable, and preferably comprises a sensor unit in particular having a position sensor.

12. Telemetry thermotransport system (10) according to any one of claims 8 to 11, **characterized in that** the container control device (16) is configured to be coupled to the remote control device (22) and/or to the transport control device (24) via a QR code and/or an NFC/RFID tag.

13. Telemetry thermotransport system (10) according to any one of claims 8 to 12, **characterized in that** the remote control device (22) is configured to configure parameters for automatic and/or remotely controllable locking and/or unlocking of the closure unit (20) in the container control device (16), in particular to configure parameters on the basis of sensor values.

## Revendications

1. Conteneur de transport (12) destiné à être utilisé dans un système de transport thermique télémétrique (10) comportant un dispositif de commande à distance (22), dans lequel sont compris une zone de réception de marchandises (14), configurable thermiquement et fermable mécaniquement, et un dispositif de commande de conteneur (16), comprenant une unité de capteurs destinée au moins à surveiller la température de la zone de réception de marchandises (14) et une unité de fermeture (20) destinée au verrouillage ou au déverrouillage commandable de la zone de réception de marchandises (14), **caractérisé en ce que** le dispositif de commande de conteneur (16) est monté de manière interchangeable dans le conteneur de transport, le dispositif de commande de conteneur (16) comprenant un accumulateur d'énergie rechargeable, de sorte que l'accumulateur d'énergie de telle sorte que l'unité de stockage d'énergie puisse être chargée séparément du conteneur de transport et que le dispositif de commande de conteneur puisse être inséré dans différents conteneurs de transport (12).

2. Conteneur de transport (12) selon la revendication 1, **caractérisé en ce qu'**au moins un élément de refroidissement et/ou de chauffage est monté de manière interchangeable pour tempérer la zone de réception de marchandises (14).

3. Conteneur de transport (12) selon l'une quelconque des revendications précédentes, **caractérisé en ce que** l'unité de capteurs comprend en outre au moins des capteurs de position, d'accélération, d'exposition à la lumière, de composition atmosphérique, de pression atmosphérique et/ou d'humidité de l'air.

4. Conteneur de transport (12) selon l'une quelconque des revendications précédentes, **caractérisé en ce que** l'unité de fermeture (20) est verrouillée ou déverrouillée au moyen d'un code QR pouvant être affiché optiquement et/ou d'un signal Bluetooth/RFID/NFC, en particulier en interaction avec un dispositif de commande de transport (24) et/ou le dispositif de commande à distance (22).

5. Conteneur de transport (12) selon l'une quelconque des revendications précédentes, **caractérisé en ce que** le dispositif de commande de conteneur (16) comprend une unité de cryptographie, de préférence une unité de cryptographie à base de clés, destinée à autoriser un verrouillage ou un déverrouillage de l'unité de fermeture (20) sur la base d'une communication de clés avec le dispositif de commande à distance (22), le dispositif de commande de transport (24) et/ou une clé côté récepteur.

6. Conteneur de transport (12) selon l'une quelconque des revendications précédentes, **caractérisé en ce que** le dispositif de commande de conteneur (16) comprend une unité de mémoire destinée à enregistrer des données de capteurs et d'actionneurs au moins depuis le verrouillage de la zone de réception de marchandises (14) jusqu'au déverrouillage ultérieur de la zone de réception de marchandises (14).

7. Conteneur de transport (12) selon l'une quelconque des revendications précédentes, **caractérisé en ce que** le dispositif de commande de conteneur (16) comprend un dispositif d'affichage destiné à l'affichage optique et/ou acoustique d'informations, en particulier d'un code QR, d'un message d'état et/ou d'un message d'avertissement.

8. Système de transport thermique télémétrique (10) destiné au transport de médicaments, de préparations pharmaceutiques ou de substances biologiquement/chimiquement sensibles, comprenant au moins un conteneur de transport (12) comportant une zone de réception de marchandises (14), configurable thermiquement et fermable mécaniquement, et un dispositif de commande de conteneur (16), comprenant une unité de capteurs (18) destinée au moins à surveiller la température de la zone de réception de marchandises et une unité de fermeture (20) destinée au verrouillage ou au déverrouillage commandable de la zone de réception de marchandises (14), et un dispositif de commande à distance (22), le dispositif de commande à distance (22) étant relié au dispositif de commande de conteneur (16) par l'intermédiaire d'un réseau afin d'interroger des données de télémétrie de capteurs de l'unité de capteurs (18) et de provoquer un verrouillage ou un déverrouillage commandable de la zone de réception de marchandises (14) par l'unité de fermeture (20), **caractérisé par** une configuration du conteneur de transport (12) selon l'une quelconque des revendications 1 à 7.

9. Système de transport thermique télémétrique (10) selon la revendication 8, **caractérisé en ce que** le réseau est un réseau sans fil, en particulier un réseau de radiotéléphonie mobile, un réseau WLAN et/ou un réseau NFC, comprenant en particulier une connexion Bluetooth ou une connexion RFID.

10. Système de transport thermique télémétrique (10) selon la revendication 8 ou 9, **caractérisé en ce qu'**un dispositif de commande de transport (24) est compris, lequel est interposé en communication ou connecté en parallèle dans le réseau entre le dispositif de commande à distance (22) et le dispositif de commande de conteneur (16), et reste spatialement adjacent au conteneur de transport (12) pendant le transport.

11. Système de transport thermique télémétrique (10) selon la revendication 10, **caractérisé en ce que** le dispositif de commande de transport (24) comprend une unité de communication par l'intermédiaire de laquelle une communication, de préférence bidirectionnelle, entre le dispositif de commande de conteneur (16) et le dispositif de commande à distance (22) est fournie, et comprend de préférence une unité de capteurs comportant en particulier un capteur de position.

12. Système de transport thermique télémétrique (10) selon l'une quelconque des revendications 8 à 11, **caractérisé en ce que** le dispositif de commande de conteneur (16) est couplé au dispositif de commande à distance (22) et/ou au dispositif de commande de transport (24) par l'intermédiaire d'un code QR et/ou d'une étiquette NFC/RFID.

13. Système de transport thermique télémétrique (10) selon l'une quelconque des revendications 8 à 12, **caractérisé en ce que** le dispositif de commande à distance (22) est conçu pour configurer, dans le dispositif de commande de conteneur (16), des paramètres destinés au verrouillage et/ou au déverrouillage automatique et/ou commandable à distance de l'unité de fermeture (20), en particulier pour configurer des paramètres sur la base de valeurs de capteurs.
